(19) Europäisches Patentamt
European Patent Office
Office européen des brevets

(11) Publication number: 0 109 818
A2

# (12) EUROPEAN PATENT APPLICATION

(21) Application number: 83306942.0

(22) Date of filing: 14.11.83

(51) Int. Cl.³: C 07 D 501/26
C 07 D 499/60

(30) Priority: 16.11.82 US 442079

(43) Date of publication of application: 30.05.84 Bulletin 84/22

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Chou, Ta-sen
745 Canyon Road
Indianapolis Indiana 46285(US)

(74) Representative: Crowther, Terence Roger et al,
Erl Wood Manor
Windlesham Surrey GU20 6PH(GB)

(54) **Process for the preparation of penicillin and cephalosporin sulfones.**

(57) The process for preparing penicillin and cephalosporin sulfones by oxidation in an aqueous medium at pH between 4.0 to 6.5 of corresponding penicillin or cephalosporin or the sulfoxide thereof with potassium hydrogen persulfate.

EP 0 109 818 A2

TITLE MODIFIED
see front page



# PROCESS FOR SULFONES

The oxidation of cephalosporin and penicillin compounds to the corresponding sulfoxides has been known for some time. The oxidation is carried out with hydrogen peroxide or a peracid, for example, perbenzoic acid, peracetic acid, or m-chloroperbenzoic acid. Little has been reported on the cephalosporin sulfones, i.e., cephalosporins fully oxidized in the 1-position. Chauvette et al. in U.S. Patent 3,536,698 teach the preparation of cephalosporin sulfones with excess amounts of the peracids employed to make the corresponding sulfoxides.

This invention relates to a process for the preparation of penicillin and cephalosporin sulfones. In particular, it relates to a process for the oxidation of penicillins and cephalosporins to the corresponding sulfones with potassium hydrogen persulfate at controlled pH.

Potassium hydrogen persulfate, also known as potassium peroxymonosulfate, is described by Kennedy et al., J. Org. Chem., 25, 1901 (1960). The oxidation of organic sulfides to sulfones, for example, thioanisole to methyl phenyl sulfone, with potassium hydrogen persulfate is described by E. M. Trost et al., Tetrahedron Lett., 22, 1287 (1981).

The process of this invention comprises the oxidation of cephalosporin and penicillin compounds or the sulfoxides thereof to the corresponding sulfones. The process is illustrated by the following partial structural formulas.

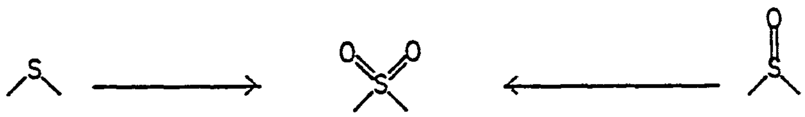

According to the invention, a compound represented by the following structural formula 1

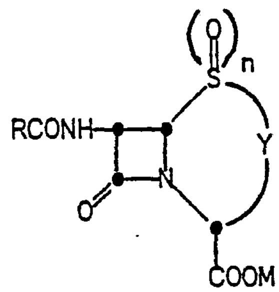 1

wherein Y is 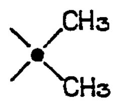 or 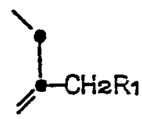

n is 0 or 1; $R_1$ is hydroxy or acetoxy; M is an alkali metal cation; and R is the residue of the acyl group RCO as defined hereinafter; is reacted in an aqueous medium maintained at a pH between about 4 and about 6.5 with potassium hydrogen persulfate to provide the corresponding sulfone represented by the following structural formula 2.

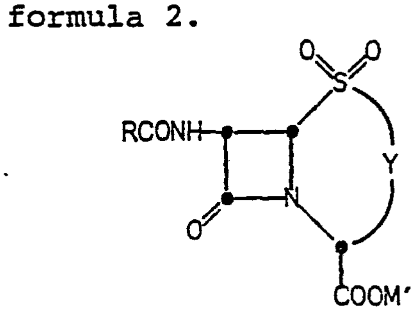

wherein R and Y have the same meanings as defined above and M' is hydrogen or an alkali metal cation.

The process is preferably carried out at a temperature between about 0°C. and about 45°C, more preferably at a temperature between about 15°C. and about 35°C.

The reaction is carried out in an aqueous medium which may comprise a water-miscible organic solvent such as a lower alcohol, for example, methyl alcohol or ethyl alcohol, at a pH of between about 4.0 and about 6.5. When the pH of the reaction medium is lower than about pH 4.0, penicillin and cephalosporin free acids are present and can precipitate from the aqueous medium. At a pH above about 6.5 the oxidant decomposes with loss of oxygen. Further, at a pH above about pH 7.0 it has been found that the 7-position side chain of the cephalosporin undergoes epimerization. For example, at a pH between about 7.5 and 8.0 the 7β-acylaminocephalosporin is epimerized to a mixture of the 7α-acylaminocephalosporin and the 7β-acylaminocephalosporin. This mixture of epimers which occurs during the oxidation at the higher pH renders the separation and crystallization of the sulfone product difficult. In carrying out the oxidation of a compound represented by the formula 1 without controlling the pH, the acidity of the reaction mixture rapidly decreases to a pH at which the penicillin or cephalosporin exists as the free acid. The latter precipitates from the aqueous medium thus preventing the desired oxidation.

Under the controlled pH conditions of the process described herein, the penicillin and cephalosporin compounds remain in solution for sulfone formation without allowing epimerization to occur.

The term R in the formula 1 represents hydrogen, $C_1$-$C_4$ alkyl, the 4-formylbutyl group $OCH(CH_2)_3$, the 4-carboxybutyl group $HOOC(CH_2)_3$, or the protected 4-carboxy-4-aminobutyl group represented by the formula

$HOOCH(CH_2)_3$
R'

wherein R' is a protected amino group; or R is group represented by the formula

R''—CH—
Q

wherein R'' is thienyl, furyl, tetrazolyl, phenyl, or a substituted phenyl group represented by the formula

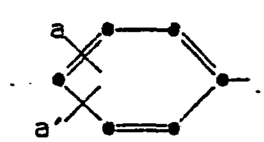

wherein a and a' independently are hydrogen, $C_1$-$C_4$ alkyl, halo, protected amino, protected aminomethyl, carboxy, carboxymethyl, hydroxy, or $C_1$-$C_4$ alkoxy; and Q is hydrogen, protected amino, hydroxy, or carboxy;

or R is a phenyl group or a substituted phenyl group represented by the formula

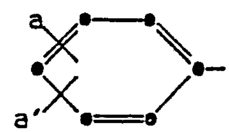

wherein a and a' have the same meanings as defined hereinabove;

or R is a phenoxymethyl group represented by the formula

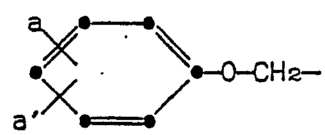

wherein a and a' are as defined above;

or R is an alkoxy or substituted alkoxy group represented by the formula

R'''-O-

wherein R''' is $C_1$-$C_5$ alkyl, $C_3$-$C_6$ cycloalkyl, adamantyl, benzyl, benzyl substituted by $C_1$-$C_4$ alkyl, $C_1$-$C_4$ alkoxy, or halo; diphenylmethyl, or diphenylmethyl substituted by $C_1$-$C_4$ alkyl, $C_1$-$C_4$ alkoxy, or halo.

As used herein, the term $C_1$-$C_4$ alkyl refers to methyl, ethyl, n-propyl, iso-propyl, n-butyl, sec-butyl, and t-butyl; $C_1$-$C_4$ alkoxy refers to methoxy, ethoxy, iso-propoxy, t-butoxy, n-butoxy, and like lower alkyl ether radicals; and halo refers to fluoro, chloro, or bromo. The term protected amino refers to a substituted amino group wherein the substituent is an art recognized protecting group readily removable to provide the free primary amino group. Examples of such groups include the alkoxycarbonyl and substituted alkoxycarbonyl-protecting groups such as ethoxycarbonyl, t-butyloxycarbonyl, trichloroethoxycarbonyl, cyclopentyloxycarbonyl, adamantyloxycarbonyl, benzyloxycarbonyl, diphenylmethoxycarbonyl, and the like; the acyl and substituted acyl groups such as acetyl, chloroacetyl, trifluoroacetyl, benzoyl, and halobenzoyl, eg. chlorobenzoyl; and trityl; and like protecting groups.

The sulfone compounds represented by the formula 1 wherein R is $HOOCCH(R')(CH_2)_3$ are derivatives of cephalosporin C ($R_1$ = acetoxy) and desacetylcephalosporin C ($R_1$ = OH). During the process of the invention, the amino group of the α-aminoadipoyl side chain is protected to prevent its oxidation by the persulfate. In addition to the amino-protecting groups described above, the amino-protecting group can be one of the groups commonly employed to protect the amino group of cephalosporin C or used as a derivative of the natural product for ease in isolation or crystallization. Since cephalosporin C itself, by its N-deacylation to 7-aminocephalosporanic acid, is used as a starting material for many semisynthetic cephalosporin antibiotics, the nature of the amino-protected group R' is not critical. Examples of R' groups are phthalimido, benzamido, chlorobenzamido, dichlorobenzamido, bromobenzamido, 2-carboxy-tetrachlorobenzamido, acetamido, chloroacetamido, propionamido, and phenylacetamido.

In the process of this invention, the cephalosporin or penicillin in free carboxylic acid form is suspended in the aqueous medium and the pH of the suspension or slurry is adjusted to between about 4 and about 6.5 by the addition of an alkali metal carbonate, bicarbonate, or hydroxide. At this pH, the carboxylic acid is converted to the soluble salt corresponding to the alkali metal employed. A solution of potassium hydrogen persulfate in water is then added to the solution of the cephalosporin or penicillin salt. In order to control the pH in the desired range, a solution of a suitable base such as an alkali metal hydroxide, carbonate, or bicarbonate, for example, sodium hydroxide, potassium hydroxide, or sodium bicarbonate is added dropwise along with the persulfate. 2N Sodium hydroxide solution is a desirable concentration of the alkali metal hydroxide for controlling the pH. Other bases which are suitably used to control pH in the process are sodium perborate monohydrate, tetrasodium phosphate, and diethylenetriamine pentaacetic acid (DTPA).

Alternatively, the acidic aqueous solution of the persulfate is treated with an alkali metal hydroxide, for example potassium hydroxide, to adjust the pH of the oxidant solution to about 5.0. To avoid decomposition of the oxidant which occurs at about pH 5 and above, the pH adjustment is carried out at ice bath temperature and the buffered oxidant solution is used promptly after preparation. This solution of the buffered oxidant is then added to the solution of the cephalosporin or penicillin salt adjusted in pH as

described above. During the addition of the buffered oxidant solution, adequate control of the pH of the oxidation reaction can be achieved by periodic addition of a 5% aqueous solution of sodium bicarbonate.

A preferred source of the persulfate oxidant is the triple salt mixture comprising 2 moles of potassium monopersulfate (potassium hydrogen persulfate, $KHSO_5$), 1 mole of potassium hydrogen sulfate, and 1 mole of potassium sulfate. This mixture is commercially available under the trade name OXONE® (monopersulfate compound) from E. I. duPont de Nemours and Company.

In the process of this invention, a cephalosporin or penicillin sulfoxide represented by the structural formula 1 wherein n is 1 is also converted to the corresponding sulfone. The sulfoxide can have either the α or the β configuration. When a cephalosporin or penicillin in the unoxidized form (sulfide form, formula 1, n = 0) is employed in the process, the sulfide form is apparently first oxidized to the sulfoxide and the latter is then further oxidized to the sulfone. In contrast to the prior methods for preparing sulfones which employ peracids such as perbenzoic acid, peracetic acid, or m-chloroperbenzoic acid, the conversion of the sulfoxide form to the sulfone occurs rapidly with ease in the process of this invention.

In an example of the process of this invention, 7β-phenylacetylaminocephalosporanic acid is suspended in water and the solution is treated with 2N

sodium hydroxide to adjust the pH to between about 5.5 and about 6.0. When the pH is adjusted, a solution of the salt form, sodium 7β-phenylacetylaminocephalosporanate, is obtained. To the solution is added an aqueous solution containing a 3 molar excess of potassium hydrogen persulfate (monopersulfate compound). The solution of the oxidant is added dropwise while at the same time a 2N solution of sodium hydroxide is also added dropwise to maintain the pH of the oxidation mixture between about 5.5 and about 6.2. When all the oxidant has been added, the reaction mixture is tested for the presence of excess oxidant, for example, with starch/KI paper. Any excess oxidant is destroyed by adding a reducing agent such as sodium bisulfite to the reaction mixture.

The sulfone product is recovered from the reaction mixture by the addition of a water-immiscible organic solvent such as ethyl acetate followed by adjustment of the pH of the mixture to about pH 2.0. The reaction mixture can be acidified with a mineral acid, for example, hydrochloric acid. The sulfone product is extracted into the organic solvent which is separated, washed, dried, and evaporated to provide the product. Alternatively, with some sulfone products provided by the process of this invention, the organic phase following washing and drying is concentrated to a small volume and the concentrate is diluted with a solvent such as diethyl ether or methylene chloride to precipitate the crystalline sulfone.

The following are illustrative of the cephalosporin sulfones provided by the process of this invention:

7-formamidocephalosporanic acid 1,1-dioxide,

7-acetamidocephalosporanic acid 1,1-dioxide,

7-adipamidocephalosporanic acid 1,1-dioxide,

7-adipamidodesacetylcephalosporanic acid 1,1-dioxide,

7-(α-phthalimidoadipamido)cephalosporanic acid 1,1-dioxide,

7-phenylacetamidocephalosporanic acid 1,1-dioxide,

7-phenoxyacetamidocephalosporanic acid 1,1-dioxide,

7-(2-thienyl)acetamidocephalosporanic acid 1,1-dioxide,

7-tetrazolylacetamidocephalosporanic acid 1,1-dioxide,

7-benzamidocephalosporanic acid 1,1-dioxide,

7-(4-methylbenzamido)cephalosporanic acid 1,1-dioxide,

7-D-(α-t-butyloxycarbamido)phenylacetamidocephalosporanic acid 1,1-dioxide,

7-D-mandelamidocephalosporanic acid 1,1-dioxide,

7-D-malonamidocephalosporanic acid 1,1-dioxide,

7-ethoxycarbonylaminocephalosporanic acid 1,1-dioxide,

7-t-butyloxycarbonylaminocephalosporanic acid 1,1-dioxide,

7-t-butyloxycarbonylaminodesacetylcephalosporanic acid 1,1-dioxide,

7-cyclopentyloxycarbonylaminocephalosporanic acid 1,1-dioxide,

7-adamantyloxycarbonylaminocephalosporanic acid 1,1-dioxide,

7-benzyloxycarbamidocephalosporanic acid 1,1-dioxide,

7-(4-chlorobenzyloxycarbamido)cephalosporanic acid 1,1-dioxide, and

7-diphenylmethoxycarbamidocephalosporanic acid 1,1-dioxide.

In carrying out the process of this invention as described hereinabove, with a compound of the formula 1 wherein $R_1$ is hydroxy, the formation of the corresponding lactone, i.e. the intramolecular ester form with the hydroxymethyl group and the carboxy group in the 4-position, can occur particularly during the acidification of the reaction mixture containing the sulfone product. Accordingly, it is desirable to carry out the isolation of the sulfone as rapidly as possible. Alternatively, the extraction of the acidified reaction mixture can be carried out with a water-immiscible organic solvent such as ethyl acetate containing an esterification reagent such as diphenyl diazomethane. The free carboxylic acid sulfone is thus converted to the corresponding ester which prevents lactone formation. Lactone formation can also be avoided by isolating the sulfone in the salt form.

In carrying out the process with a cephalosporin compound the preferred pH is between about 5.0 and about 6.5.

In a further example of the process, potassium 6β-phenoxyacetamidopenicillanate is dissolved in water and cooled to ice bath temperature. A solution of potassium hydrogen persulfate containing at least a 3 molar excess and adjusted to pH 5.6 is added dropwise with stirring to the cold penicillin salt solution. The pH of the oxidation mixture is maintained at between about 4.0 to about 5.0 by the periodic addition of an aqueous solution of sodium bicarbonate. After all of the oxidant is added, the reaction mixture is allowed to stir at room temperature to insure complete sulfone formation. Excess persulfate is destroyed by the addition of a reducing agent such as sodium bisulfite. The penicillin sulfone product is isolated in either of two ways. In one, the reaction mixture is acidified to pH 2 to 2.5 with an acid such as hydrochloric acid. Some of the penicillin sulfones will precipitate as the free acids directly from the aqueous acidified reaction mixture, particularly when the volume of the reaction mixture is kept low. Alternatively, the reaction mixture prior to acidification is layered with a water immiscible organic solvent in which the penicillin sulfone is soluble. The mixture is then acidified to about pH 2.0 to 2.5 and the sulfone free acid is extracted into the organic solvent. The extract is separated, washed, dried and evaporated to provide the product.

Preferably, in the oxidation of a penicillin or a sulfoxide thereof the pH is controlled between about 4.0 and about 5.5.

The following are examples of penicillin sulfones provided by the process of this invention:

6-formamidopenicillanic acid 1,1-dioxide,

6-acetamidopenicillanic acid 1,1-dioxide,

6-adipamidopenicillanic acid 1,1-dioxide (penicillin N sulfone),

6-phenylacetamidopenicillanic acid 1,1-dioxide (penicillin G sulfone),

6-phenoxyacetamidopenicillanic acid 1,1-dioxide (penicillin V sulfone),

6-(4-methylbenzamido)penicillanic acid 1,1-dioxide,

6-benzamidopenicillanic acid 1,1-dioxide,

6-D-phenylglycylaminopenicillanic acid 1,1-dioxide (ampicillin sulfone),

6-D-malonamidopenicillanic acid 1,1-dioxide (carbenacillin sulfone),

6-D-mandelamidopenicillanic acid 1,1-dioxide,

6-D-[2-carboxy-2-(2-thienyl)acetamido]penicillanic acid 1,1-dioxide,

6-t-butyloxycarbamidopenicillanic acid 1,1-dioxide,

6-ethoxycarbamidopenicillanic acid 1,1-dioxide,

6-adamantyloxycarbamidopenicillanic acid 1,1-dioxide,

6-cyclopentyloxycarbamidopenicillanic acid 1,1-dioxide,

6-benzyloxycarbamidopenicillanic acid 1,1-dioxide, and

6-diphenylmethoxycarbamidopenicillanic acid 1,1-dioxide.

The cephalosporin sulfones provided by the process of this invention are useful starting materials in a process for preparing 1-oxa-β-lactam antibiotics. The conversion of these sulfone products to the 1-oxa antibiotic compounds is a multi-step process comprising the epimerization of the 7β-acylaminocephalosporin sulfone to the corresponding 7α-acylamino sulfone; deacetylation (formula 1), $R_1$ = acetoxy) to the 7α-acylamino-3-hydroxymethyl sulfone; electrolysis of the epimeric 3-hydroxymethyl sulfone to a 3α-acylamino azetidin-2-one-4-sulfinic acid; and the cyclization of the azetidinone sulfinic acid to a 3-methyl-1-oxa-β-lactam and the isomeric 3-exomethylene 1-oxa compound.

The foregoing sequence of reactions employing the cephalosporin sulfones obtained by the process of this invention is illustrated in the following reaction scheme.

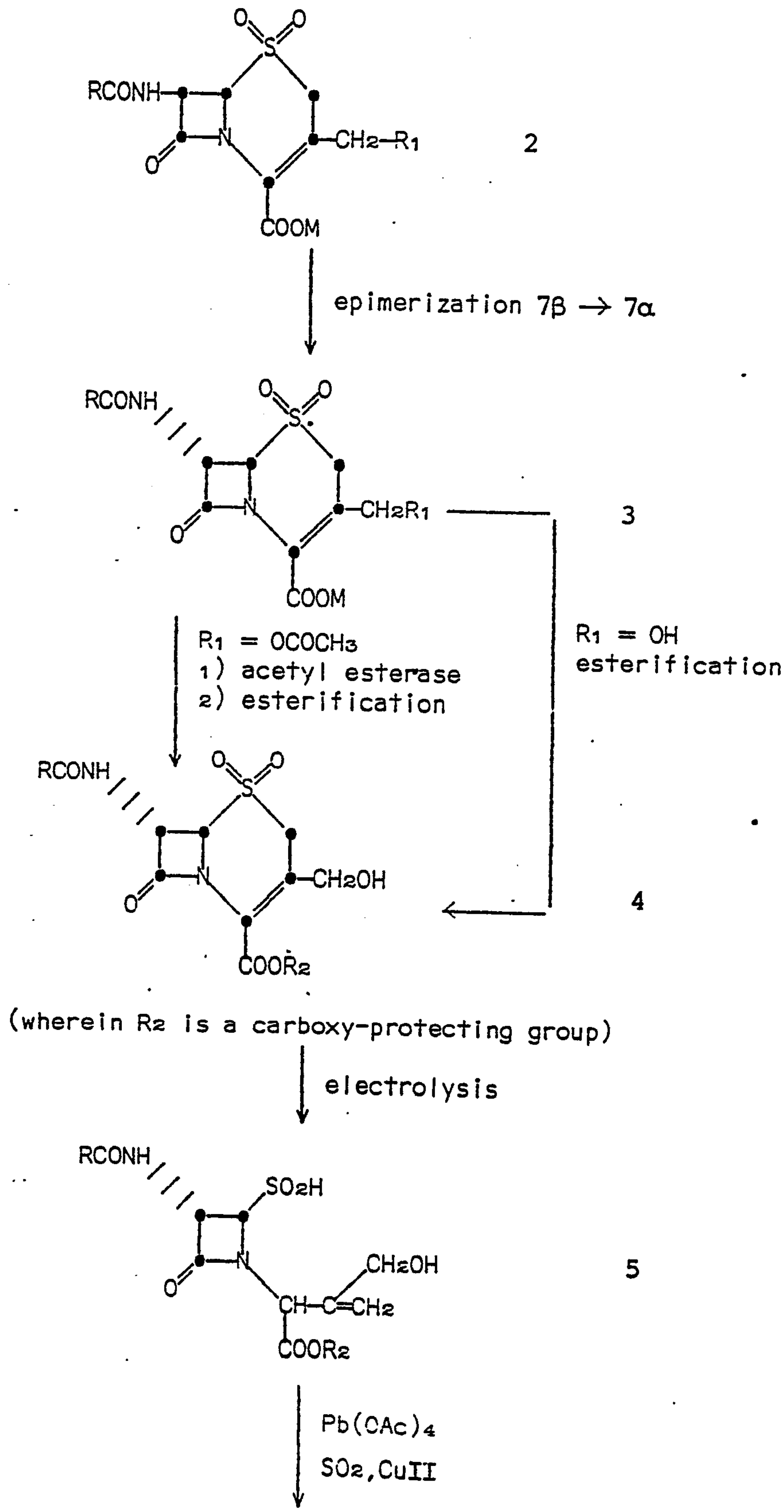


2

3

4

(wherein $R_2$ is a carboxy-protecting group)

5

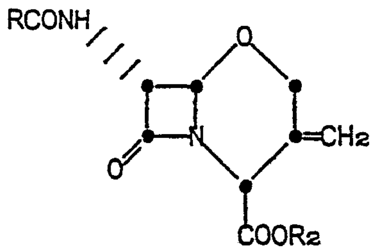 + 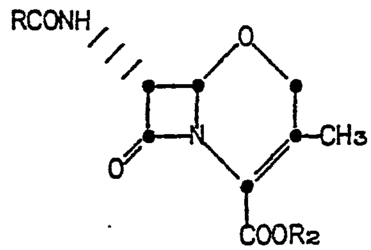 6

The epimerization of the sulfone free acid to the 7α-acylamino sulfone is preferably carried out in an aqueous medium as follows. A slurry of the sulfone free acid in water is treated with an aqueous solution containing at least an equimolar amount of sodium acetate. An aqueous solution of piperazine is then added dropwise until the pH of the solution is adjusted to between about 9.5 to about 10. With the pH adjusted, the epimerization mixture is stirred for about 15 minutes and the epimer is recovered as follows. A water-insoluble organic solvent such as an ester, for example, ethyl acetate, is added to the epimerization solution which is then acidified to a pH of about 2.0. The epimeric 7α-acylamino sulfone free acid is then extracted with the organic solvent.

The epimeric sulfone is deacylated with acetyl esterase to provide the epimeric 3-hydroxymethylcephalosporin sulfone. The deacetylation is preferably carried out with the esterase immobilized on a modified silica gel.

The immobilization of the acetyl esterase is carried out as follows. Silica gel of 70-230 mesh and 62-200 mμ particle size is cleaved by heating a slurry of the silica in aqueous 10% nitric acid at a temperature of about 80°C. for 3 hours. After cooling,

the silica is washed thoroughly with water. The cleaned silica gel is then slurried in 10% 3-aminopropyltriethoxysilane and the slurry deaerated under vacuum. The pH of the slurry is adjusted to from about 3 to about 4 with dilute hydrochloric acid and the acidified slurry is heated at 80°C. for about 3 hours with periodic agitation. The modified silica is filtered, washed with water, and dried for about 16 hours at 105°C. The dry silica is then slurried with aqueous 3% glutaraldehyde buffered with pH 7 phosphate buffer (5-10 vol./wt. of silica). This slurry is periodically agitated for 3 hours and is then washed with water and pH 7 citrate buffer. This modified silica gel can then be packed into a column or, alternatively, in another suitable vessel and a neutral aqueous solution of the acetyl esterase is added to the resin and allowed to interact for about 20 hours. The silica/enzyme is then transferred to a glass column and washed with pH 7 citrate buffer.

The cephalosporin sulfone represented by formula 1 ($R_1$ is acetoxy) is dissolved in 0.2 M aqueous sodium citrate and the pH of the solution adjusted to 7 with 1N sodium hydroxide. The solution is then passed over the immobilized enzyme in the column and the effluent collected. Ethyl acetate is added to the effluent and the mixture is chilled to 0°C. The pH of the cold aqueous organic mixture is adjusted to about 2.5 with hydrochloric acid and the ethyl acetate layer is separated. The acidified aqueous phase is further extracted with ethyl acetate and all extracts are combined and washed with acidified brine and then

dried. The 3-hydroxymethyl cephalosporin sulfone is recovered from the ethyl acetate by evaporation. Alternatively, the epimeric desacetylcephalosporin sulfone can be esterified at this stage. For example, the washed and dried ethyl acetate extract of the effluent is concentrated under reduced pressure and the concentrate treated with the esterifying reagent, for example, diphenyldiazomethane, to form the desired ester, for example, the diphenylmethyl ester.

The esterified 7α-acylamino-3-hydroxymethylcephalosporin sulfone is then reduced at the cathode of an electrolysis cell comprising a mercury pool cathode, a platinum anode, and a cationic resin bridge. Suitable electrolytes are, for example, sodium perchlorate, lithium chloride, or sodium acetate. The anolyte is preferably phosphate buffer, pH 2.7.

The reduction is carried out preferably at a temperature between about -10°C. and about 10°C. at a reduction potential between about -1.9 v. and about 1.9 v. The 3-hydroxymethyl sulfone ester is dissolved in methyl alcohol containing a proton source such as acetic acid and an electrolyte salt such as sodium acetate is added to the solution until a concentration of 0.1N with respect to the electrolyte is achieved. The solution is then added to the cathode compartment of the electrolysis cell which is then flushed with argon to remove oxygen. The electrolysis is then carried out either at constant potential or at constant current. The progress of the electrolysis can be monitored by use of analytical HPLC carried out on an aliquot of the reduction mixture.

The above procedures are described in more detail in copending applications reference Nos. X-5017, X-5510, X-4998 of even date herewith.

Following the electrolysis, the reduction product mixture is extracted in the cold with a water-immiscible organic solvent, preferably ethyl acetate. The 3-epiazetidin-2-one-4-sulfinic acid (formula 5 above) can be purified by HPLC.

The azetidinone sulfinic acid, 5, is then cyclized to an isomeric mixture of the 3-exomethylene and corresponding 3-methyl-1-oxa-β-lactam compound represented by formula 6 in the above reaction scheme. The cyclization is carried out according to the process described in co-pending application reference No. X-5321 of even date herewith. According to the described process, the azetidinone sulfinic acid is reacted at a temperature between about -25°C. and about 0°C. with between about 1 and about 2.5 mole equivalents of lead tetraacetate in a dipolar aprotic solvent such as sulfur dioxide and preferably in the presence of copper II ion. The reaction proceeds rapidly on a mole scale, i.e. in an hour or less. The reaction proceeds rapidly on a mole scale, i.e. in an hour or less. The reaction product mixture is then diluted with a water-immiscible organic solvent such as ethyl acetate and the mixture is washed with brine and pH 7 phosphate buffer. The organic solvent containing the products is separated and washed further before drying. Evaporation of the solvent provides the isomeric mixture of products which can be separated on reverse phase $C_{18}$ silica gel, HPLC.

The 1-oxa-β-lactam cyclized products, 6, obtained with the cephalosporin sulfones of this invention as described hereinabove are themselves useful intermediates for the preparation of known 1-oxa-β-lactam antibacterial compounds. For example, the 7α-acylamino-3-methyl or 3-exomethylene carboxylic acid in esterified form can be methoxylated in the 7-position with conversion of the 7-epi side chain to the natural or 7β-configuration. The methoxylation is carried out according to the procedure described by Narisada et al., U.S. Patent No. 4,138,486. The methoxylated product is a 7β-acylamino-7α-methoxy substituted 1-oxa-β-lactam ester. The 7α-methoxylated 3-exomethylene-1-oxa-β-lactam compounds obtained in the above-described methoxylation are useful as intermediates in the preparation of 3-alkoxy and 3-halo substituted compounds. For example, the 7β-acylamino-7α-methoxy-3-exomethylene ester is reacted with ozone to form the 3-hydroxy ester and the latter can be reacted with diazomethane to form the corresponding 3-methoxy compound. Alternatively, the 3-hydroxy compound can be reacted with phosphorus trichloride and dimethylformamide to form the 3-chloro ester. Following these reactions at the 3-position, the 7-position side chain can be removed by known N-deacylation procedures and the 7-amino-3-substituted 1-oxa-β-lactam nucleus reacylated with a derivative of the appropriate carboxylic acid to provide the desired compound in esterified form. Following the acylation, any amino or carboxy-protecting groups can be removed to provide the

desired antibacterial compound in the free acid form. The N-deacylation can be achieved by either of two known methods. For example, when in the formula 1 R is an alkoxy or substituted alkoxy group, the 7-position side chain is an alkoxycarbamido group, for example ethoxycarbamido, or a substituted alkoxy group, for example benzyloxycarbamido. Treatment of the cyclized product with trifluoroacetic acid effects the removal of the alkoxycarbonyl or substituted alkoxycarbonyl side chain and provides the 7-amino deacylation product. The benzyloxycarbonyl and substituted benzyloxycarbonyl and like side chains can also be removed by catalytic hydrogenolysis over 5% Pd/C. Alternatively, when the side chain in the cyclized product is an acylamino group, for example phenylacetylamino, the cyclized product is reacted with phosphorus pentachloride in an inert halogenated hydrocarbon solvent such as di or trichloroethane to provide the imino chloride of the 7-position amido group. The imino chloride is converted to an imino ether by treating the imino chloride with a lower alcohol or a benzyl alcohol, and the imino ether upon hydrolysis provides the 7-amino 3-substituted 1-oxa-β-lactam nucleus compound.

The cephalosporin sulfones provided by the process of this invention are also useful in the preparation of 7β-[2-(2-aminothiazol-4-yl)-2-methoxyiminoacetamido]cephalosporanic acid 1,1-dioxide having antibiotic properties. W. Dürckheimer in "Recent Advances in the Chemistry of β-Lactam Antibiotics", Special Publication No. 38, G. I. Gregory, Ed., The

Royal Soc. of Chem., Burlington House, London, 1980, Chapter 4. 7-Aminocephalosporanic acid 1,1-dioxide in esterified form is obtained by the N-deacylation procedures described hereinabove, and is reacylated with an active derivative of 2-(2-aminothiazol-4-yl)-2-methoxyiminoacetic acid to provide, following deesterification, the antibiotic compound.

The penicillin sulfones provided by the process of this invention, like other penicillin sulfones, are useful to a greater or lesser degree in inhibiting β-lactamase enzymes produced by microorganisms. As such they can be employed in assays for determining the amount of β-lactam antibiotics present in a sample by preventing their enzymatic destruction. The penicillin sulfones may also be used as adjuncts to β-lactam antibiotics by retarding antibiotic destruction during therapy.

The following examples further illustrate the process of this invention.

Example 1

7β-(4-Methylbenzamido)cephalosporanic acid 1,1-dioxide

To a slurry of 156 g. of 7β-(4-methylbenzamido)-cephalosporanic acid in 400 ml. of water were added 660 ml. of 5% sodium bicarbonate and 1 ml. of Anti Foam A. The final pH of the solution was 5.9.

A solution of 592 g. of potassium hydrogen persulfate (pH 2.0) in 2.2 liters of water was treated with 324 ml. of 4N potassium hydroxide to adjust the pH of the oxidant solution to 4.9.

The oxidant solution was added to the solution of the cephalosporin at room temperature. During the addition, 5% sodium bicarbonate was added from time to time to maintain the pH of the reaction mixture between 5.2 and 5.9. During the addition of the oxidant solution which took about 45 min., the temperature of the reaction mixture rose to 32°C.

The reaction mixture was stirred for two hours after which time the thin layer chromatogram run with a small aliquot showed only product in the mixture. The excess potassium hydrogen persulfate was destroyed by the addition of sodium bisulfite. The pH of the reaction mixture was 1.8 and the sulfone product began to gum out of solution. The reaction mixture was extracted with 3 l. of ethyl acetate, the extract washed with water, dried over magnesium sulfate, filtered, and concentrated in a rotary evaporator. The residue of crude product was dissolved in 300 ml. of methylene chloride containing 200 ml. of ethyl acetate. The product, 7β-(4-methylbenzamido)cephalosporanic acid 1,1-dioxide, crystallized from solution. There were obtained 83.3 g., 49.3% yield, of first crop material.

Example 2

The above oxidation was repeated on a larger scale using 313.5 g. of the same starting material and with the pH maintained between 5.5 and 5.9. There were obtained 206.4 g. of first crop sulfone and 34.9 g. of second crop sulfone for a combined yield of 241.3 g. (76.9%).

Example 3

7β-Phenylacetamidocephalosporanic acid 1,1-dioxide

A suspension of 8.128 g. of 7β-phenylacetamidocephalosporanic acid in 30 ml. of water was treated with enough 2N sodium hydroxide until the pH was adjusted to between 5.5 and 6.2 and a solution obtained. A solution of 29.6 g. of potassium hydrogen persulfate in 110 ml. of water was added dropwise to the cephalosporin solution, while at the same time 2N sodium hydroxide was added dropwise to maintain the pH of the mixture between 5.5 and 6.2. Addition required 45 min. and the mixture was stirred for one hour post addition. Sodium bisulfite (solid) was then added to destroy excess oxidant. Ethyl acetate (50 ml.) was added and the pH of the mixture was adjusted to about 2.0 with concentrated hydrochloric acid. The ethyl acetate extract was separated and the mixture extracted two more times with fresh 50 ml. portions of ethyl acetate. The extracts were combined, washed with water, dried over magnesium sulfate, filtered, and concentrated by evaporation to a volume of about 50 ml. The concentrate was diluted with an equal volume of diethyl ether and allowed to stand in the refrigerator. The product crystallized and was filtered. There were obtained 2.414 g. of first crop material and 0.925 g. of second crop product. The combined crops represented a 39.5% yield of sulfone product.

## Example 4

7β-Phenoxyacetamidocephalosporanic acid 1,1-dioxide

A suspension of 78.2 g. (0.183 mole) of 7β-phenoxyacetamidocephalosporanic acid in 250 ml. of water was treated with 5% sodium bicarbonate until the pH of the solution was 5.9 and a solution was obtained. A solution of 300 g. of potassium hydrogen persulfate in 1.2 l. of water (pH adjusted to 4.2 with 4NKOH) was added dropwise to the cephalosporin solution, while at the same time a 5% solution of sodium bicarbonate was added dropwise to maintain the pH of the mixture between 5.2 and 5.9. Addition of the oxidant was completed in 45 min. after the reaction mixture was stirred for 35 min. at room temperature, 50 ml. of ethyl acetate were added, and the pH of the mixture was adjusted to 1.8 with concentrated hydrochloric acid. The ethyl acetate was separated, washed with water, dried over magnesium sulfate, filtered, and concentrated by evaporation to a volume of about 250 ml. The concentrate was diluted with an equal volume of diethyl ether and allowed to stand in the refrigerator. The sulfone product crystallized from the cold solution. There were obtained 7.41 g. of first crop product. The mother liquor was evaporated to yield 35 g. of additional sulfone product as a non-crystalline foam.

Example 5

7β-Benzyloxycarbamidocephalosporanic acid 1,1-dioxide

A suspension of 40.64 g. (0.1M) of 7β-benzyloxycarbamidocephalosporanic acid in 150 ml. of water was treated with about 55 ml. of 2N sodium hydroxide to adjust the pH of about 5.5 to 6.2. At this pH the cephalosporanic acid went into solution.. The solution was treated by dropwise addition with a solution of 148 g. of potassium hydrogen persulfate in 550 ml. of water. Simultaneously, 2N sodium hydroxide was added dropwise to maintain the pH of the oxidation mixture at about 5.5 to 6.2. After the additions were complete the mixture was stirred for one hour. The mixture was positive for excess oxidant (KI-starch paper) and about 1.5 g. of sodium bisulfite were added to destroy the excess.

Ethyl acetate (ca. 200 ml.) was added to the mixture and the pH was adjusted to 2.0 with concentrated hydrochloric acid. The ethyl acetate was separated and the acidified mixture was extracted twice again with ethyl acetate. The extracts were combined, washed with water, dried over magnesium sulfate, filtered, and evaporated to dryness. The residue of sulfone product was dissolved in 50 ml. of a 1:1, v:v, mixture of methylene chloride and diethyl ether and the solution was cooled in the refrigerator for 24 hours. When the product failed to crystallize, the solution was evaporated to dryness and the residue of the sulfone product was dissolved in a 1:2, v:v, mixture of ethyl

acetate and diethyl ether. The solution was allowed to stand in the refrigerator for several days for crystallization to occur. There were obtained 6.336 g. of the white crystalline sulfone product.

## Example 6

Disodium 7β-[4-(2,4-dichlorobenzamido)-4-carboxyvaleramido]cephalosporanate 1,1-dioxide

To a solution of 100 g. (0.16 mole) of the disodium salt of 7β-[4-(2,4-dichlorobenzamido)-4-carboxyvaleramido]cephalosporanic acid in 400 ml. of water was added dropwise with stirring a solution of 194 g. of potassium hydrogen persulfate ($2KHSO_5 \cdot KHSO_4 \cdot K_2SO_4$) in 800 ml. of water. The pH of the persulfate solution was adjusted to 2.4 with 5N sodium hydroxide prior to addition. As the persulfate solution was added, the pH of the reaction mixture was maintained at about 5.7 by the dropwise addition of a saturated solution of sodium bicarbonate. The formation of the sulfone product was monitored by reverse phase $C_{18}$ silica HPLC using 28% acetonitrile -0.5% acetic acid-water. After the reaction was complete (1 hr. 50 min.), 16.6 g. of sodium bisulfite were added to the reaction mixture to reduce excess oxidant. The reaction mixture (total volume ca. 1800 ml.) was layered with 1400 ml. of ethyl acetate and the mixture was chilled to a temperature of about 8°C. in an ice-alcohol bath. The pH of the cold mixture was adjusted to 1.5 with 12N sulfonic acid. After agitation, the ethyl acetate layer was separated. The aqueous acidic phase was extracted twice with 1 l.

portions of ethyl acetate each and the extracts combined. The extracts were dried over magnesium sulfate, filtered, and diluted with fresh ethyl acetate to a volume of about 3300 ml. To the ethyl acetate solution of the sulfone product was added a solution of 52 g. of sodium 2-ethylhexanoate in 350 ml. of ethyl acetate. The product precipitated as a very fine precipitate which plugged the filter on filtration. After standing overnight, the product was filtered successfully and 105 g. of the sulfone were obtained.

Example 7

7β-t-Butyloxycarbamidocephalosporanic acid 1,1-dioxide

The pH of a suspension of 7β-t-butyloxycarbamidocephalosporanic acid is adjusted to 6.0 with 2N sodium hydroxide to form a solution of the sodium salt. To the solution is added dropwise an aqueous solution containing an excess of potassium hydrogen persulfate. Throughout the addition of the oxidant the pH of the reaction mixture is maintained between about 6.0 and about 6.2 by the dropwise addition of 1N sodium hydroxide. After all of the persulfate solution has been added, sodium bisulfite is added to reduce excess oxidant. The mixture is then layered with ethyl acetate and acidified to pH 2 with 1N hydrochloric acid with stirring. The ethyl acetate layer is separated, washed with water, dried and evaporated to provide the sulfone product.

Example 8

6β-Phenoxyacetamidopenicillanic acid 1,1-dioxide

A suspension of 139.0 g. of the salt mixture 2KHSO5·$KHSO_4$·$K_2SO_4$ in 500 ml. of water was warmed to form a solution which then was cooled in an ice bath to about 0-5°C. The pH of the cold solution was adjusted to 5.6 with 2N sodium hydroxide and a solution of 39.0 g. of potassium 6β-phenoxyacetamidopenicillanate in 200 ml. of water was added dropwise. During the addition of the penicillin salt solution the pH of the reaction mixture was maintained at 4.0 to 5.0 by the periodic addition of an aqueous 5% solution of sodium bicarbonate. Addition of the penicillin salt solution was completed in 30 min. During the addition the reaction mixture was kept cold in an ice bath. After addition was completed, the reaction mixture was warmed to a temperature of about 40-45°C. and was stirred at this temperature for 3.5 hours at pH 4-5. Sodium bisulfite was added to the reaction mixture until the mixture gave a negative test with starch iodide paper. As the bisulfite was being added the pH of the solution dropped to 2.6 and the penicillin sulfone product began to precipitate as white crystals. The pH was lowered to 2.0 with concentrated hydrochloric acid and the mixture was stirred overnight at ice bath temperature to complete crystallization. The product was filtered and air dried at 50°C. There were obtained 25.0 g. of 6β-phenoxyacetamidopenicillanic acid as white crystals melting at about 101 to about 104°C. Mass spec. M/e 382.

A second crop of product, 350 mg., precipitated from the filtrate.

CLAIMS

1. A process for preparing a sulfone of the formula

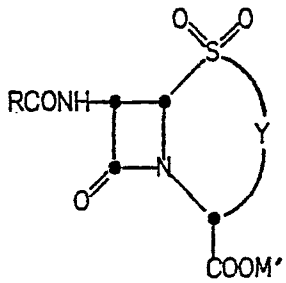

which comprises mixing in an aqueous medium at a pH between about 4 to about 6.5 a compound of the formula

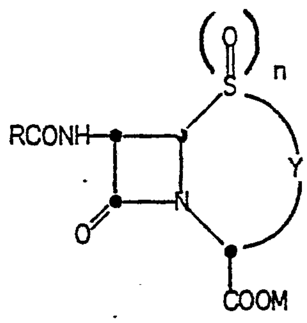

with potassium hydrogen persulfate, where

Y is 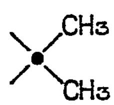 or 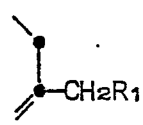 ;

n is 0 or 1;

R is hydrogen, $C_1$-$C_4$ alkyl, $OCH(CH_2)_3$ , $HOOC(CH_2)_3$ ,

$HOOC-CH(CH_2)_3$
R'

wherein R' is a protected amino group;

or R is a group of the formula

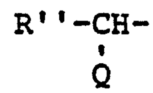

wherein R'' is thienyl, furyl, tetrazolyl, phenyl, or a substituted phenyl group of the formula

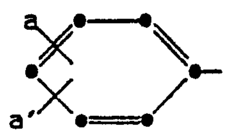

wherein a and a' independently are hydrogen, $C_1$-$C_4$ alkyl, halo, protected amino, protected aminomethyl, carboxy, carboxymethyl, carbamoyl, $C_1$-$C_4$ alkoxy, or hydroxy; Q is hydrogen, protected amino, hydroxy, or carboxy;

or R is a phenyl group or a substituted phenyl group of the formula

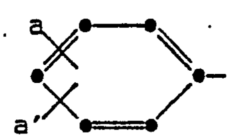

wherein a and a' have the same meanings as defined hereinabove;

or R is a group of the formula

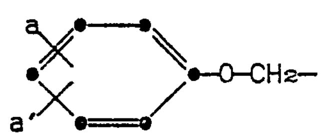

wherein a and a' are as defined hereinabove; or R is an alkoxy or substituted alkoxy group of the formula

R'''-O-

wherein R''' is $C_1$-$C_5$ alkyl, $C_3$-$C_6$ cycloalkyl, adamantyl, benzyl, benzyl substituted by $C_1$-$C_4$ alkyl, $C_1$-$C_4$ alkoxy, or halo; diphenylmethyl, or diphenylmethyl substituted by $C_1$-$C_4$ alkyl, $C_1$-$C_4$ alkoxy, or halo;

$R_1$ is acetyl or hydroxy;

M is an alkali metal cation; and M' is hydrogen or an alkali metal cation.

2. The process of claim 1 wherein Y is

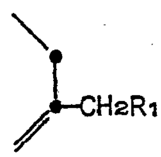

3. The process of claim 1 or 2 wherein R is

R'''-O-

4. The process of claim 1, 2 or 3 wherein R''' is benzyl, $R_1$ is acetoxy, and n is 0.

5. The process of claim 1 or 2 wherein R is a phenyl group of the formula

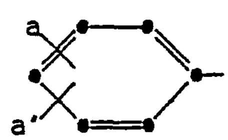

6. The process of claim 1 or 2 wherein R is 4-methylphenyl, $R_1$ is acetoxy, and n is 0.

7. The process of any one of the preceding claims wherein the pH is between about 5.0 to 6.0.

8. The process of claim 1 or 2 wherein R is

R''-CH-
|
Q .

9. The process of claim 8 wherein R'' is

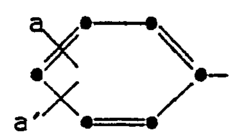

Q is hydrogen, n is 0, and $R_1$ is acetoxy.

10. The process of claim 8 wherein 7β-phenylacetamidocephalosporanic acid sodium salt is reacted with potassium hydrogen sulfate at a pH between about 5.5 and about 6.5.